(19) Europäisches Patentamt
European Patent Office
Office européen des brevets

(11) **EP 3 188 708 B1**

(12) **EUROPEAN PATENT SPECIFICATION**

(45) Date of publication and mention
of the grant of the patent:
**03.05.2023 Bulletin 2023/18**

(21) Application number: **15763175.5**

(22) Date of filing: **02.09.2015**

(51) International Patent Classification (IPC):
*A61K 8/29* (2006.01)        *A61K 8/895* (2006.01)
*A61K 8/02* (2006.01)        *A61Q 17/04* (2006.01)

(52) Cooperative Patent Classification (CPC):
**A61K 8/29; A61K 8/0241; A61K 8/895; A61Q 17/04**

(86) International application number:
**PCT/US2015/048098**

(87) International publication number:
**WO 2016/036828 (10.03.2016 Gazette 2016/10)**

(54) **MINERAL SUNSCREEN COMPOSITIONS**

**MINERALISCHE SONNENSCHUTZZUSAMMENSETZUNGEN**

**COMPOSITIONS D'ÉCRAN SOLAIRE MINÉRAL**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **02.09.2014 US 201462044490 P**

(43) Date of publication of application:
**12.07.2017 Bulletin 2017/28**

(60) Divisional application:
**23159115.7**

(73) Proprietor: **Edgewell Personal Care Brands, LLC St. Louis MO 63017 (US)**

(72) Inventors:
• **LI, Geng**
  **Rutherford, New Jersey 07070 (US)**
• **SANOGUEIRA, James**
  **Wesley Hills, New York 10901 (US)**

(74) Representative: **dompatent von Kreisler Selting Werner -
Partnerschaft von Patent- und Rechtsanwälten mbB
Deichmannhaus am Dom
Bahnhofsvorplatz 1
50667 Köln (DE)**

(56) References cited:
**WO-A1-2014/101698        US-A1- 2009 175 915
US-A1- 2013 052 147        US-B1- 8 545 891**

**Description**

BACKGROUND OF THE INVENTION

A. Field of Endeavor

The present invention relates to mineral sunscreen compositions with self-leveling and self-spreading properties, and to methods of using the composition to protect keratinous substrates such as skin and hair against ultraviolet radiation.

B. Background Information

Topical photoprotective agents, commonly known as sunscreens, must address two factors in order to provide effective sun protection. First, it should provide broad spectrum uniform protection against ultraviolet (UV) radiation in the UVA region (320 to 400 nm) and UVB region (290 to 320 nm). The UVA region is further divided between the shorter wavelengths of UVA-II radiation from 320 to 340 nm, and the slightly longer wavelengths of UVA-I radiation from 340 to 400 nm. Protection from UVA radiation is measured as a Protection Factor - UVA or PFA. Protection from UVB radiation is measured as a Sun Protection Factor or SPF.

The second factor for effective sun protection is user compliance. The sunscreen should have a pleasing sensory and tactile profile to enhance the user experience and promote compliance in use. For optimum sun protection, a user needs to re-apply the sunscreen every two hours. Sunscreen compositions that contain physical particles, namely mineral sunscreens, also referred to as sunblocks, containing titanium dioxide and/or zinc oxide are often thick, pasty, and have an unpleasant heavy feel during application. After application, they form an undesirable whiteness on the skin. These factors do result in diminished user compliance. While mineral sunscreens provide uniform protection against UV radiation well into the UVA-I region to fulfill the critical wavelength requirement of the FDA monograph more readily than organic sunscreens using a single sunscreen active, sun protection is insufficient if the sensory experience is a barrier to user compliance.

Thus, it is desirable to achieve a sunscreen composition using one or more mineral sunscreen actives that provides consumers with an improved sensory experience that provides enhanced sun protection against both UVA and UVB rays.

US8545891 discloses sunscreen compositions comprising a combination of titanium dioxide filtering agents and film forming agents in emulsion.

SUMMARY

In a first aspect, the present invention is directed to a sunscreen composition according to claim 1, comprising an anhydrous combination comprising (i) titanium dioxide and (ii) one or more film forming polymers, wherein the sunscreen composition has a spreading value of at least about 10 cm$^2$ at 20 seconds without rub out. The sunscreen composition may also have a spreading value of at least about 18 cm$^2$ at 20 seconds without rub out. Preferably, the sunscreen composition is substantially emulsion-free. Preferably, the titanium dioxide may be present in an amount of up to about 40.0 wt. %; more preferably, the titanium dioxide may be present in an amount of up to about 30.0 wt. %; and most preferably, the titanium dioxide may be present in an amount of up to about 20.0 wt.%. Preferably, the one or more film formers are selected from the group consisting of acrylate copolymers, polyester copolymers, polyolefin copolymers, silicone copolymers, and combinations thereof. More preferably, the one or more film formers may comprise acrylate copolymers. the sunscreen composition further includes a diluent. The diluent may be selected from the group consisting of isododecane, dodecane, tridecane, isohexadecane, pentadecane, and combinations thereof. More preferably, the diluent comprises isododecane.

Preferably, the sunscreen composition may further include an additional sunscreen agent. The additional sunscreen agent may comprise zinc oxide. The additional sunscreen agent may also comprise one or more organic sunscreen agents selected from the group consisting of *p*-aminobenzoic acid and derivatives thereof; butyl methoxydibenzoylmethane; benzophenones; hydroxy-substituted benzophenones; methoxy-substituted benzophenones; benzophonone-1; benzophenone-2; benzophenone-3; benzophenone-4; benzophenone-6; benzophenone-8; benzophenone-12; methoxycinnamate; ethyl dihydroxypropyl-*p*-aminobenzoate; glyceryl-*p*-aminobenzoate; homosalate; methyl anthranilate; octocrylene; octyl dimethyl-*p*-aminobenzoate; octyl methoxycinnamate; octyl salicylate; 2 phenylbenzimidazole-5-sulphonic acid; triethanolamine salicylate; 3-(4-methylbenzylidene)-camphor; red petrolatum,3-(4-methylbenzyldine)boran-2-one(methylbenzindinecamphor); benzotriazole; salicylates; phenylbenzimidazole-5-sulfonic acid; methylene *bis*-benzotriazolyl tetramethylbutyl phenol; avobenzone; 4-isopropyldibenzoylmethane; butylmethoxydibenzoylmethane; octisalate; oxybenzone; *bis*-ethylhexyloxyphenol methoxy triazine; 4-isopropyl-dibenzoylmethane; metal oxides; zinc oxide; octyltriethoxy silanol; alumina; triethoxy silane; and combinations thereof. Preferably, the additional sunscreen agent may comprise one or more organic sunscreen agents selected from the group consisting of octocrylene, homosalate, octisalate, and combinations thereof.

In another aspect, the present invention is directed to a sunscreen according to claim 1, consisting essentially of a synergistic combination comprising (i) titanium dioxide present in an amount of up to about 40.0 wt. %; (ii) a film former comprising acrylate copolymers, polyester copolymers, polyolefin copolymers, silicone copolymers, or combinations thereof; and (iii) a diluent, wherein the sunscreen composition has a spreading value of at least about 10 cm$^2$ at 20 seconds without rub out. Preferably, the sunscreen composition has a spreading value of at least about 20 cm$^2$ at 20 seconds without rub out. Preferably, the film former may comprise cyclopentasiloxane (and) acrylate/dimethicone copolymers. More preferably, the film former is present in an amount of about 0.05 wt. % to about 5.0 wt. %. Most preferably, the film former is present in an amount of about 1.0 wt. % to about 2.0 wt. %. Preferably, the titanium dioxide is present in an amount of up to about 20.0 wt. %. More preferably, the sunscreen composition may further include zinc oxide. Preferably, the diluent is selected from the group consisting of isododecane, dodecane, tridecane, isohexadecane, pentadecane, and combinations thereof.

In yet another aspect, the present invention is directed to a sunscreen composition consisting essentially of (i) a synergistic combination comprising a therapeutic amount of titanium dioxide to provide an SPF of about 80 to about 200, and a film former comprising acrylate copolymers, polyester copolymers, polyolefin copolymers, silicone copolymers, or combinations thereof; (ii) a diluent; and (iii) an additional sunscreen agent, wherein the sunscreen composition has a spreading value of at least about 20 cm$^2$ at 20 seconds without rub out.

In still yet another aspect, the present invention is directed to a consumer packaged product comprising a sunscreen composition of any of the embodiments disclosed herein.

In still yet another aspect, the present invention is directed to a method of protecting hair from ultraviolet radiation by applying any sunscreen composition disclosed herein to the keratinous substrate.

In still yet another aspect, the present invention is directed to a method of absorbing ultraviolet radiation by applying any sunscreen composition disclosed herein to a keratinous substrate and subjecting the keratinous substrate to ultraviolet radiation.

DETAILED DESCRIPTION OF THE INVENTION

The embodiments of the present invention can comprise, consist of, and consist essentially of the features and/or steps of the invention described herein, as well as any of the additional or optional ingredients, components, steps, or limitations described herein or would otherwise be appreciated by one of skill in the art. It is to be understood that all concentrations disclosed herein are by weight percent (wt. %) based on a total weight of the composition unless otherwise indicated. Where appropriate, the INCI (International Nomenclature of Cosmetic Ingredients) name of ingredients/components is used.

The present invention is directed to a sunscreen composition containing a mineral sunscreen agent, which has improved spreadability due to its self-leveling properties, provides a light and silky feel on a keratinous substrate, and unexpectedly fulfills the FDA Critical Wavelength test method to achieve a critical wavelength of ≥370 nm for broad spectrum protection. The sunscreen composition includes the following: an anhydrous combination comprising a therapeutic amount of a mineral sunscreen agent such as titanium dioxide and a film former. Preferably, the composition also includes a diluent. It may further comprise a variety of additional components to provide additional functionality to the user. The unexpected synergy of the mineral sunscreen agent, preferably titanium dioxide, and the film former provides broad spectrum protection against UVB through UVA-I radiation up to and beyond the critical wavelength of 370 nm when used alone or in combination with additional sunscreen agents. The sunscreen compositions of the present invention can be formulated with pharmaceutically acceptable carrier components to provide different formulations as a lotion or spray product. A further embodiment provides a consumer packaged product wherein a porous applicator can be used to apply the sunscreen composition on a keratinous substrate surface by a user.

Titanium dioxide useful in the aforementioned sunscreen compositions is preferably available as an anhydrous slurry or paste in a hydrocarbon carrier. The titanium dioxide particles themselves typically have an inert coating to reduce its photocatalytic activity. Some titanium dioxide particles have one or more coatings. Preferably, the titanium dioxide particles useful in the disclosed formulation have at least one coating comprising silicone copolymers; fatty acids or salts thereof; oxides of silicon, aluminum, zirconium, and the like; and combinations thereof. By choosing a coating that is compatible with the carrier, the resulting formulation enjoys enhanced stability and the titanium dioxide particles remain well dispersed in the sunscreen composition with no particle settling.

Preferred embodiments utilize titanium dioxide sold under the trade name SiClone® TD-150 by Presperse Corporation, Somerset, New Jersey. The SiClone TD-150 product contains about 40% titanium dioxide (solids content) with an inner coating of aluminum hydroxide and an outer coating of isostearic acid in a hydrocarbon carrier (isohexadecane/isododecane/C$_{13}$ to C$_{15}$ alkanes) that is substantially free of any wetting agents. The titanium dioxide is preferably present in a therapeutically effective amount to provide an SPF of greater than 77 and a critical wavelength of at least 371 nm; more preferably in an amount of about 15.0 wt. % to about 40.0 wt. %, and more preferably in an amount of about 15.0 wt. % to about 20.0 wt. %, all based on a total weight of the sunscreen composition.

The film formers useful in the anhydrous combination of the sunscreen composition disclosed herein provide a boosting effect in the SPF of the titanium dioxide. The SPF of the titanium dioxide, neat SiClone TD-150, is about 77. By adding a preferred film former, the SPF is boosted by about 6% to about 80% or more exhibiting a synergistic effect between the preferred film former and the titanium dioxide since the film former itself does not register an SPF. When an additional sunscreen agent is added into the formulation, the SPF can be further increased by about 250% or more depending on the particular additional sunscreen agent and amounts thereof. In a most preferred embodiment, the SPF is boosted by the addition of the film former by at least about 6%, and in some embodiments by at least about 80%.

Preferred film formers can be selected from the group consisting of acrylate copolymers, polyester copolymers, polyolefin copolymers, and combinations thereof. Exemplary acrylate copolymers are silicone acrylates such as, but not limited to, cyclopentasiloxane (and) acrylate/dimethicone copolymer; acrylic acid/isobornyl methacrylate/isobutyl methacrylate copolymer/isododecane; isododecane (and) acrylate/dimethicone copolymer; and combinations thereof. Exemplary of a polyester copolymer useful in the present disclosure is octyldodecyl citrate crosspolymer. Exemplary of polyolefin polymers useful in the present disclosure are isododecane (and) butylene/ethylene/propylene copolymer; and hydrogenated polycyclopentadiene (and) isododecane. The above lists are only examples and non-limiting.

Most preferred film formers comprise acrylate copolymers. Commercially available silicone acrylate copolymers are sold under the trade name KP-545 from Shinetsu Corporation, Japan; GIOVAREZ® AC-34M from Phoenix Chemical, Inc. of Somerville, New Jersey; and CHEMSIL® K-50 from ChemSil Silicones, Inc. of Chatsworth, California. The film former can be present in an amount sufficient to boost the SPF of the titanium dioxide by at least 6% and, in some cases, over 80%, and up to 250% is also possible. Preferably, the film former is present in an amount of about 0.05 wt. % to about 5.0 wt. %, based on a total weight of the sunscreen composition. More preferably, the film former is present in an amount of about 1.0 wt. % to about 3.0 wt. %, based on a total weight of the sunscreen composition. Most preferably, the film former is present in an amount of up to about 1.0 wt. % to about 2.0 wt. %, based on a total weight of the sunscreen composition.

The sunscreen composition may further include a chemically compatible non-aqueous diluent. Preferably the diluent is a hydrocarbon diluent. It can be selected from the group consisting of isododecane, dodecane, tridecane, isohexadecane, pentadecane, and combinations thereof. Preferred diluents are isododecane; isohexadecane/isododecane/$C_{13}$ to $C_{15}$ alkane; pentadecane; dodecane/tridecane. The above lists are only examples and are not limiting. Commercially available diluents are sold under the trade name PERMETHYL® 99A and SiClone SR5, both from Presperse Corporation; and ELEVANCE® Soft from Elevance Renewable Sciences, Inc.

The diluent is preferably present in a quantity sufficient (q.s.) to bring the total amount of titanium dioxide and film former to 100.0 g. Depending on the final composition, the diluent may be present in an amount of up to about 95.0 wt. %, more preferably based on an amount of about 25.0 wt. % to about 95.0 wt. %, and most preferably based on an amount of about 25.0 wt. % to about 50.0 wt. %, all based on a total weight of the sunscreen composition.

The sunscreen composition of the present disclosure may further include additional sunscreen agents such as zinc oxide or organic sunscreen agents, alone or in combination. Organic sunscreen agents that can be used in the sunscreen composition include, but are not limited to *p*-aminobenzoic acid and derivatives thereof; butyl methoxydibenzoylmethane; benzophenones; hydroxy-substituted benzophenones; methoxy-substituted benzophenones; benzophenone-1; benzophenone-2; benzophenone-3; benzophenone-4; benzophenone-6; benzophenone-8; benzophenone-12; methoxycinnamate; ethyl dihydroxypropyl-*p*-aminobenzoate; glyceryl-*p*-aminobenzoate; homosalate; methyl anthranilate; octocrylene; octyl dimethyl-*p*-aminobenzoate; octyl methoxycinnamate; octyl salicylate; 2 phenylbenzimidazole-5-sulphonic acid; triethanolamine salicylate; 3-(4-methylbenzylidene)-camphor; red petrolatum,3-(4-methylbenzyldine)boran-2-one(methylbenzindinecamphor); benzotriazole; salicylates; phenylbenzimidazole-5-sulfonic acid; methylene *bis*-benzotriazolyl tetramethylbutyl phenol; avobenzone; 4-isopropyldibenzoylmethane; butylmethoxydibenzoylmethane; octisalate; oxybenzone; *bis*-ethylhexyloxyphenol methoxy triazine; 4-isopropyl-dibenzoylmethane; metal oxides; zinc oxide; octyltriethoxy silanol; alumina; triethoxy silane; and combinations thereof.

The preferred additional sunscreen agents are zinc oxide, avobenzone, octyl salicylate, homosalate, octocrylene, or combinations thereof. The one or more sunscreen agents can be included in a present composition at about 5.0 wt. % to about 35.0 wt. % based on a total weight of the composition. The amount and types of sunscreen agents in the composition will vary in the above range depending on the target SPF. The higher the SPF, the greater the total amount of sunscreen agents. Preferably, the one or more additional sunscreen agents are present in an amount of about 3.0 wt. % to about 30.0 wt. % to achieve a SPF of about 80 to about 200 and more, based on a total weight of the sunscreen composition. More preferably, the one or more additional sunscreen agents are present in an amount of about 3.0 wt. % to about 25.0 wt. %, based on a total weight of the sunscreen composition.

The cosmetic compositions of the present invention may optionally include other active or inactive ingredients such as those selected from, but not limited to, cosmetically acceptable carriers, oils, sterols, amino acids, moisturizers, powders, colorants (including pigments and/or dyes), pH adjusters, perfumes, essential oils, cosmetic active ingredients, vitamins, essential fatty acids, sphingolipids, self-tanning compounds such as dihydroxyacetone (DHA) and erythruloses, fillers, emulsifying agents, antioxidants, surfactants, additional film formers, chelating agents, gelling agents, thickeners, emollients, humectants, moisturizers, minerals, viscosity and/or rheology modifiers, keratolytics, retinoids, hormonal compounds, alpha-hydroxy acids, alpha-keto acids, anti-mycobacterial agents, anti-fungal agents, antimicrobial, anti-virals, analgesics, anti-allergenic agents, H1 or H2 antihistamines, antiinflammatory agents, anti-irritants, anti-neoplastics, immune system boosting agents, immune system suppressing agents, anti-acrie agents, anesthetics, antiseptics, insect repellents, skin cooling compounds, skin protectants, skin penetration enhancers, exfoliants, lubricants, fragrances, colorants, staining agents, depigmenting agents, hypopigmenting agents, preservatives, stabilizers, pharmaceutical agents, photostabilizing agents, spherical powders, one or more fragrances, plant extracts, absorbents, salicylic acid, alpha and beta hydroxy acids, vitamins including vitamins A, C, and E, retinol and its derivatives, or any mixtures thereof. The above is a list of examples and is not meant to be limiting.

In some embodiments, the sunscreen agent is titanium dioxide wherein the particles have an inner coating of aluminum hydroxide and an outer coating of isostearic acid, the film former is cyclopentasiloxane (and) acrylate/dimethicone copolymer, and the diluent is isododecane.

In some embodiments, the sunscreen agent is titanium dioxide wherein the particles have an inner coating of aluminum hydroxide and an outer coating of isostearic acid, the film former is acrylic acid/isobornyl methacrylate/isobutyl methacrylate copolymer/isododecane, and the diluent is isododecane.

In some embodiments, the sunscreen agent is titanium dioxide wherein the particles have an inner coating of aluminum hydroxide and an outer coating of isostearic acid, the film former is cyclopentasiloxane (and) acrylate/dimethicone copolymer, and the diluent is isohexarie/isododecane/$C_{13}$ to $C_{15}$ alkane.

In some embodiments, the sunscreen agent is titanium dioxide wherein the particles have an inner coating of aluminum hydroxide and an outer coating of isostearic acid, the film former is cyclopentasiloxane (and) acrylate/dimethicone copolymer, and the diluent is pentadecane.

In some embodiments, the sunscreen agent is titanium dioxide wherein the particles have an inner coating of aluminum hydroxide and an outer coating of isostearic acid, the film former is cyclopentasiloxane (and) acrylate/dimethicone copolymer, and the diluent is dodecane and tridecane.

In some embodiments, the sunscreen agents are zinc oxide and titanium dioxide wherein the titanium dioxide particles have an inner coating of aluminum hydroxide and an outer coating of isostearic acid, the film former is cyclopentasiloxane (and) acrylate/dimethicone copolymer, and the diluent is isododecane.

In some embodiments, the sunscreen agent is titanium dioxide wherein the particles have an inner coating of aluminum hydroxide and an outer coating of isostearic acid, the film former is isododecane (and) acrylate/dimethicone copolymer, and the diluent is isododecane.

In some embodiments, the sunscreen agent is titanium dioxide wherein the particles have an inner coating of aluminum hydroxide and an outer coating of isostearic acid, the film former is cyclopentasiloxane (and) acrylate/dimethicone copolymer, the diluent is isododecane, and the additional sunscreen agents are homosalate, octisalate, octocrylene, and avobenzone.

The sunscreen compositions according to the present disclosure may be prepared according to techniques that are well known to one of ordinary skill in the art. The present disclosure will be better understood from the examples that follow, all of which are intended for illustrative purposes only and are not meant to limit the scope of the instant disclosure in any way.

Samples of the sunscreen composition of the present disclosure shown in Table I were prepared using the following method: to a clean and sanitized beaker were placed an amount of titanium dioxide slurry (SiClone TD-150 as a dispersion with about 40% solids content) and an amount of a film former. The titanium dioxide and film former were mixed together at about 70°C for about 20 minutes. The mixture was homogenized with stirring at 2500 rpm for about 10 minutes then cooled to about 40°C to which an amount of diluent was added. Again the mixture was homogenized with stirring at 2500 rpm until the mixture reached room temperature. In samples containing organic sunscreens agents, the organic sunscreens agents were mixed together in a separate vessel and heated to about 75°C until a clear mixture (neat) was obtained. The clear mixture of organic sunscreen agents was then added to the heated titanium dioxide mixture prior to homogenization and cooling to room temperature.

Preferably, the sunscreen composition of the present invention comprises a single anhydrous phase such that emulsifiers are not needed in formulating the inventive composition, nor is a multi-phase process required in a method of making the inventive composition.

Samples were prepared using 50.0 wt. % of the SiClone TD-50, which provided a solids content of about 20.0 wt. % to about 25 wt. % of titanium dioxide particles unless otherwise indicated in Table I; 0.05 wt. % to 5.0 wt. % film former was used unless otherwise indicated, and the specified diluent was used in a quantity sufficient to bring the weight of the composition to 100.0 g. Samples 8 to 10 have additional sunscreen agents. Sample 12 is a comparative example of a mineral sunscreen agent comprising only 5.0 wt. % zinc oxide (ZnO).

| TABLE I | | | |
|---|---|---|---|
| Sample No. | Film Former* | Diluent | Notes |
| 1 | A | isododecane | |
| 2 | B | isododecane | |
| 3 | B | isohexane/isododecane/ $C_{13}$-$C_{15}$ alkane | |
| 4 | B | pentadecane | |
| 5 | B | dodecane/tridecane | |
| 6 | C | isododecane | |
| 7 | B | -- | approx. 40%$TiO_2$ in dispersion |
| 8 | A | isododecane | +3 wt. % to 25 wt. % organic sunscreen mix of homosalate, octisalate, octocrylene, avobenzone |
| 9 | A | isododecane | 2 wt. % film former |
| 10 | A | isododecane | 3 wt. % film former |
| 11 | A | isododecane | + 5 wt. % ZnO |
| 12 | A | isododecane | No $TiO_2$; only sunscreen agent is 5 wt. % ZnO |
| *Film former A: GIOVAREZ AC-34M; Film former B: KP-545; Film Former C: CHEMSIL K-50 | | | |

While additional additives may be added, they are not required. As such, the sunscreen compositions of the present disclosure are incredibly simple to manufacture with fewer ingredients that may be sensitizing to consumers.

*In vitro* SPF and critical wavelength ($\lambda_c$) data were obtained using a Labsphere Ultraviolet Transmittance Analyzer (Model UV-2000 available from the Solar Light Company, Philadelphia, Pennsylvania) and presented in TABLE II. Samples of the sunscreen compositions weighing 1.6 mg/cm$^2$ were transferred by an adjustable pipette and uniformly applied to a Schonberg sand-blast PMMA plate (roughness 6 $\mu$m) by finger with a presaturated finger cot. After application, the coated plate was air dried for 15 minutes. The sample plate was then placed inside the Labsphere Analyzer. Irradiation took place at 4 randomly selected points. The readings were recorded by the analyzer and the calculation of the SPF value was based on the following equation:

$$ SPF = \frac{\int_{290nm}^{400nm} E_\lambda \cdot S_\lambda \cdot d\lambda}{\int_{290nm}^{400nm} E_\lambda \cdot S_\lambda \cdot T_\lambda \cdot d\lambda} $$

with the use of built-in software: UV-2000 application Version 1.1.0.0, wherein $E_\lambda$ is defined as the Commission Internationale de l'Eclairage (CIE) erythemal spectral effectiveness, $S_\lambda$ is designated as the solar spectral irradiance, and $T_\lambda$ is the spectral transmittance of the sample as measured on the UV-2000. An average of four readings was recorded as the in-vitro SPF value of each sample shown in Table II.

| TABLE II | | |
|---|---|---|
| Sample No. | SPF | $\lambda_c$ |
| 1 | 89 | 372 |
| 2 | 91 | 371 |
| 3 | 93 | 367 |
| 4 | 91 | 367 |

(continued)

| TABLE II | | |
|---|---|---|
| Sample No. | SPF | $\lambda_c$ |
| 5 | 93 | 367 |
| 6 | 82 | 371 |
| 7 | 139 | 370 |
| 8 | 193 | 378 |
| 9 | 95 | 372 |
| 10 | 92 | 373 |
| 11 | 104 | 370 |
| 12 | 3 | 377 |

The sunscreen compositions of Samples 1 to 11 show an increase in SPF when compared to the SPF of the neat SiClone TD-150 without any film former suggesting there is a boosting effect when the titanium dioxide is formulated with a selected film former in accordance with the present disclosure. In Samples 1 and 2, the SPF was boosted about 18% while also meeting the critical wavelength requirement. Sample 11 incorporates 5.0 wt. % of an additional sunscreen agent into the formulation, namely zinc oxide, to provide an even higher SPF than using zinc oxide alone as in Sample 12 that included the film former and diluent.

Spreading values of each of the above samples were determined by applying a 0.5 g sample to the back of a subject's hand without rubbing, and measuring the spread area after 20 seconds. Results are presented in TABLE III with comparative examples (Samples A, B, C, and D) also shown.

| TABLE III | |
|---|---|
| Sample No. | Spreading Value (cm$^2$) |
| 1 | 20 |
| 2 | 20 |
| 3 | 18 |
| 4 | 20 |
| 5 | 24 |
| 6 | 20 |
| 7 | 10 |
| 8 | 18 |
| 9 | 20 |
| 10 | 20 |
| 11 | 18 |
| 12 | 20 |
| A | 2 |
| B | 2 |
| C | 10 |
| D | 10 |
| Sample A: Commercial Product containing 20.0 wt. % ZnO only<br>Sample B: Commercial Product containing 5.0 wt. % TiO$_2$ and 3.0 wt. % ZnO<br>Sample C: SiClone TD-150 neat containing 40.0% solids<br>Sample D: Commercial Product containing 42.0 wt. % TiO$_2$ only | |

Inventive Samples 1 to 6 and 8 to 12 demonstrate the exceptional self-spreading properties of the mineral sunscreen composition of the permanent invention when the sunscreen composition is formulated with the film formers disclosed herein. By incorporating one or more of the selected film formers into the mineral sunscreen formulation, the spreading values show about a ten-fold increase over the commercially available mineral sunscreen products, Samples A and B, making the inventive sunscreen composition easier to spread on the skin without tugging and leaving a light, silky finish.

The sunscreen compositions of the present invention can be used in a consumer packaged product as a lotion or formulated as a spray product. The viscosity of the sunscreen compositions is less than about 1000 cPs, and preferably less than 750 cPs. Given the low viscosity of the sunscreen composition, a spray product can be a finger pump product or a continuous spray product. The consumer packaged product may also comprise a porous plastic applicator. Such a product may contain a reservoir for storing the composition and a wick to draw the sunscreen composition from the reservoir to the applicator for easy application by a user.

A method of protecting hair would comprise applying an amount of a sunscreen composition of the present invention to the substrate by a user. A method of absorbing ultraviolet radiation would comprise applying an amount of a sunscreen composition of the present invention by a user to a keratinous substrate and subjecting the keratinous substrate to ultraviolet radiation. The present disclosure also concerns a composition according to claim 1 for use in the protection of skin against ultraviolet radiation.

Sunscreen compositions containing mineral sunscreen agents, titanium dioxide and zinc oxide, are known to be thick and pasty. They are difficult to spread leaving a heavy, undesirable skin feel with a white chalky appearance after application. However, by utilizing one or more of the specific film formers disclosed herein, sunscreen compositions comprising titanium dioxide can have a light, silky finish and a synergistic effect is shown wherein the SPF, and preferably also the PFA, of the titanium dioxide is increased. The addition of an additional sunscreen agent such as zinc oxide and other organic sunscreen agents can provide further broad spectrum protection to keratinous substrates against damaging UV radiation.

**Claims**

1. A sunscreen composition comprising an anhydrous combination comprising

    - titanium dioxide particles,
    - one or more film forming polymers and
    - a diluent

    wherein said sunscreen composition has a spreading value of at least 10 cm$^2$ at 20 seconds without rub out, wherein the spreading value is determined by applying a 0.5 g sample of the sunscreen composition to the back of a subject's hand without rubbing, and measuring the spread area after 20 seconds, wherein said sunscreen composition is emulsion-free and does not contain an emulsifier and wherein:

    (i) the titanium dioxide particles have an inner coating of aluminum hydroxide and an outer coating of isostearic acid, the film former is cyclopentasiloxane (and) acrylate/dimethicone copolymer, and the diluent is isododecane; or

    (ii) the titanium dioxide particles have an inner coating of aluminum hydroxide and an outer coating of isostearic acid, the film former is acrylic acid/isobornyl methacrylate/isobutyl methacrylate copolymer/isododecane, and the diluent is isododecane; or

    (iii) the titanium dioxide particles have an inner coating of aluminum hydroxide and an outer coating of isostearic acid, the film former is cyclopentasiloxane (and) acrylate/dimethicone copolymer, and the diluent is isohexane/isododecane/C$_{13}$ to C$_{15}$ alkane; or

    (iv) the titanium dioxide particles have an inner coating of aluminum hydroxide and an outer coating of isostearic acid, the film former is cyclopentasiloxane (and) acrylate/dimethicone copolymer, and the diluent is pentadecane; or

    (v) the titanium dioxide particles have an inner coating of aluminum hydroxide and an outer coating of isostearic acid, the film former is cyclopentasiloxane (and) acrylate/dimethicone copolymer, and the diluent is dodecane and tridecane; or

    (vi) the sunscreen agents are zinc oxide and titanium dioxide particles, wherein the titanium dioxide particles have an inner coating of aluminum hydroxide and an outer coating of isostearic acid, the film former is cyclopentasiloxane (and) acrylate/dimethicone copolymer, and the diluent is isododecane; or

    (vii) the titanium dioxide particles have an inner coating of aluminum hydroxide and an outer coating of isostearic acid, the film former is isododecane (and) acrylate/dimethicone copolymer, and the diluent is

8

isododecane; or

(viii) the titanium dioxide particles have an inner coating of aluminum hydroxide and an outer coating of isostearic acid, the film former is cyclopentasiloxane (and) acrylate/dimethicone copolymer, the diluent is isododecane, and the additional sunscreen agents are homosalate, octisalate, octocrylene, and avobenzone.

2. The sunscreen composition of claim 1, wherein said titanium dioxide is present in an amount of up to 40.0 wt. %, more preferably up to 30.0 wt. % and most preferably up to 20.0 wt. %.

3. The sunscreen composition of any of claims 1 or 2 further including an additional sunscreen agent selected from zinc oxide and one or more organic sunscreen agents.

4. The sunscreen composition of claim 3, wherein said one or more organic sunscreen agent is selected from the group consisting of p-aminobenzoic acid and derivatives thereof; butyl methoxydibenzoylmethane; benzophenones; hydroxy-substituted benzophenones; methoxy-substituted benzophenones; benzophonone-1; benzophenone-2; benzophenone-3; benzophenone-4; benzophenone-6; benzophenone-8; benzophenone-12; methoxycinnamate; ethyl dihydroxypropyl-*p*-aminobenzoate; glyceryl-*p*-aminobenzoate; homosalate; methyl anthranilate; octocrylene; octyl dimethyl-*p*-aminobenzoate; octyl methoxycinnamate; octyl salicylate; 2 phenylbenzimidazole-5-sulphonic acid; triethanolamine salicylate; 3-(4-methylbenzylidene)-camphor; red petrolatum; 3-(4-methylbenzyldine)boran-2-one(methylbenzindinecamphor); benzotriazole; salicylates; phenylbenzimidazole-5-sulfonic acid; methylene *bis*-benzotriazolyl tetramethylbutyl phenol; avobenzone; 4-isopropyldibenzoylmethane; octisalate; oxybenzone; *bis*-ethylhexyloxyphenol methoxy triazine; octyltriethoxy silanol; triethoxy silane; and combinations thereof.

5. The sunscreen composition of any of claims 1 to 4, wherein said film former is present in an amount of 0.05 wt. % to 5.0 wt. %.

6. The sunscreen composition of any of claims 1 or 2 further including an additional sunscreen agent.

7. An apparatus comprising a porous applicator portion and a wick and the sunscreen composition as defined in any of claims 1 to 6.

8. A consumer-packaged product comprising the sunscreen composition of any of claims 1 to 6.

9. Use of the sunscreen composition according to any of claims 1 to 6 for protecting hair against ultraviolet radiation.

10. The sunscreen composition of any of claims 1 to 6 for use in the protection of skin against ultraviolet radiation.

**Patentansprüche**

1. Sonnenschutzzusammensetzung, umfassend eine wasserfreie Kombination, umfassend

- Titandioxidteilchen,
- ein oder mehrere filmbildende Polymere und
- ein Verdünnungsmittel,
wobei die Sonnenschutzzusammensetzung einen Ausbreitungswert von wenigstens 10 cm$^2$ nach 20 Sekunden ohne Ausreiben aufweist, wobei der Ausbreitungswert dadurch bestimmt wird, dass man eine 0,5-g-Probe der Sonnenschutzzusammensetzung auf den Handrücken eines Probanden aufträgt, ohne zu reiben, und nach 20 Sekunden die Ausbreitungsfläche misst,
wobei die Sonnenschutzzusammensetzung emulsionsfrei ist und keinen Emulgator enthält und wobei:

(i) die Titandioxidteilchen eine innere Beschichtung aus Aluminiumhydroxid und eine äußere Beschichtung aus Isostearinsäure aufweisen, es sich bei dem Filmbildner um Cyclopentasiloxan (und) Acrylat/Dimethicon-Copolymer und bei dem Verdünnungsmittel um Isododecan handelt; oder

(ii) die Titandioxidteilchen eine innere Beschichtung aus Aluminiumhydroxid und eine äußere Beschichtung aus Isostearinsäure aufweisen, es sich bei dem Filmbildner um Acrylsäure/Isobornylmethacrylat/Isobutylmethacrylat-Copolymer/Isododecan und bei dem Verdünnungsmittel um Isododecan handelt; oder

(iii) die Titandioxidteilchen eine innere Beschichtung aus Aluminiumhydroxid und eine äußere Beschichtung aus Isostearinsäure aufweisen, es sich bei dem Filmbildner um Cyclopentasiloxan (und) Acrylat/Dimethicon-Copolymer und bei dem Verdünnungsmittel um Isohexan/Isododecan/C$_{13}$- bis C$_{15}$-Alkan handelt; oder

(iv) die Titandioxidteilchen eine innere Beschichtung aus Aluminiumhydroxid und eine äußere Beschichtung aus Isostearinsäure aufweisen, es sich bei dem Filmbildner um Cyclopentasiloxan (und) Acrylat/Dimethicon-Copolymer und bei dem Verdünnungsmittel um Pentadecan handelt; oder

(v) die Titandioxidteilchen eine innere Beschichtung aus Aluminiumhydroxid und eine äußere Beschichtung aus Isostearinsäure aufweisen, es sich bei dem Filmbildner um Cyclopentasiloxan (und) Acrylat/Dimethicon-Copolymer und bei dem Verdünnungsmittel um Dodecan und Tridecan handelt; oder

(vi) es sich bei den Sonnenschutzmitteln um Zinkoxid- und Titandioxidteilchen handelt, wobei die Titandioxidteilchen eine innere Beschichtung aus Aluminiumhydroxid und eine äußere Beschichtung aus Isostearinsäure aufweisen, es sich bei dem Filmbildner um Cyclopentasiloxan (und) Acrylat/Dimethicon-Copolymer und bei dem Verdünnungsmittel um Isododecan handelt; oder

(vii) die Titandioxidteilchen eine innere Beschichtung aus Aluminiumhydroxid und eine äußere Beschichtung aus Isostearinsäure aufweisen, es sich bei dem Filmbildner um Isododecan (und) Acrylat/Dimethicon-Copolymer und bei dem Verdünnungsmittel um Isododecan handelt; oder

(viii) die Titandioxidteilchen eine innere Beschichtung aus Aluminiumhydroxid und eine äußere Beschichtung aus Isostearinsäure aufweisen, es sich bei dem Filmbildner um Cyclopentasiloxan (und) Acrylat/Dimethicon-Copolymer und bei dem Verdünnungsmittel um Isododecan handelt und die zusätzlichen Sonnenschutzmittel Homosalat, Octisalat, Octocrylen und Avobenzon sind.

2. Sonnenschutzzusammensetzung gemäß Anspruch 1, wobei das Titandioxid in einer Menge von bis zu 40,0 Gew.-%, besonders bevorzugt bis zu 30,0 Gew.-% und am meisten bevorzugt bis zu 20,0 Gew.-% vorhanden ist.

3. Sonnenschutzzusammensetzung gemäß einem der Ansprüche 1 oder 2, weiterhin umfassend ein zusätzliches Sonnenschutzmittel, das aus Zinkoxid und einem oder mehreren organischen Sonnenschutzmitteln ausgewählt ist.

4. Sonnenschutzzusammensetzung gemäß Anspruch 3, wobei das eine oder die mehreren organischen Sonnenschutzmittel aus der Gruppe ausgewählt sind, die aus *p*-Aminobenzoesäure und Derivaten davon; Butylmethoxydibenzoylmethan; Benzophenonen; hydroxysubstituierten Benzophenonen; methoxysubstituierten Benzophenonen; Benzophenon-1; Benzophenon-2; Benzophenon-3; Benzophenon-4; Benzophenon-6; Benzophenon-8; Benzophenon-12; Methoxycinnamat; Ethyldihydroxypropyl-*p*-aminobenzoat; Glyceryl-*p*-aminobenzoat; Homosalat; Methylanthranilat; Octocrylen; Octyldimethyl-*p*-aminobenzoat; Octylmethoxycinnamat; Octylsalicylat; 2-Phenylbenzimidazol-5-sulfonsäure; Triethanolaminsalicylat; 3-(4-Methylbenzyliden)campher; roter Vaseline; 3-(4-Methylbenzylidin)boran-2-on (Methylbenzidincampher); Benzotriazol; Salicylaten; Phenylbenzimidazol-5-sulfonsäure; Methylen-*bis*-benzotriazolyltetramethylbutylphenol; Avobenzon; 4-Isopropyldibenzoylmethan; Octisalat; Oxybenzon; Bis(ethylhexyloxyphenol)methoxytriazin; Octyltriethoxysilanol; Triethoxysilan und Kombinationen davon besteht.

5. Sonnenschutzzusammensetzung gemäß einem der Ansprüche 1 bis 4, wobei der Filmbildner in einer Menge von 0,05 Gew.-% bis 5,0 Gew.-% vorhanden ist.

6. Sonnenschutzzusammensetzung gemäß einem der Ansprüche 1 oder 2, weiterhin umfassend ein zusätzliches Sonnenschutzmittel.

7. Vorrichtung, umfassend einen porösen Applikatorteil und einen Docht und die Sonnenschutzzusammensetzung gemäß einem der Ansprüche 1 bis 6.

8. Fertig verpacktes Erzeugnis, umfassend die Sonnenschutzzusammensetzung gemäß einem der Ansprüche 1 bis 6.

9. Verwendung der Sonnenschutzzusammensetzung gemäß einem der Ansprüche 1 bis 6 zum Schützen von Haaren vor Ultraviolettstrahlung.

10. Sonnenschutzzusammensetzung gemäß einem der Ansprüche 1 bis 6 zur Verwendung beim Schützen von Haut vor Ultraviolettstrahlung.

**Revendications**

1. Composition d'écran solaire comprenant une combinaison anhydre, comprenant

- des particules de dioxyde de titane,
- un ou plusieurs polymères filmogènes, et
- un diluant,

dans laquelle ladite composition d'écran solaire présente une valeur d'expansion d'au moins 10 cm$^2$ à 20 secondes sans frottage, où la valeur d'expansion est déterminée en appliquant un échantillon de 0,5 g de la composition d'écran solaire sur le dos de la main d'un sujet sans frotter, et en mesurant la superficie d'expansion après 20 secondes,

dans laquelle ladite composition d'écran solaire est exempte d'émulsion et ne contient pas d'émulsifiant, et dans laquelle :

(i) les particules de dioxyde de titane présentent un revêtement intérieur d'hydroxyde d'aluminium, et un revêtement extérieur d'acide isostéarique, ledit agent filmogène est le cyclopentasiloxane (et) un copolymère d'acrylate/diméthicone, et le diluant est l'isododécane, ou

(ii) les particules de dioxyde de titane présentent un revêtement intérieur d'hydroxyde d'aluminium, et un revêtement extérieur d'acide isostéarique, ledit agent filmogène est un copolymère d'acide acrylique/méthacrylate d'isobornyle/méthacrylate d'isobutyle/isododécane, et le diluant est l'isododécane, ou

(iii) les particules de dioxyde de titane présentent un revêtement intérieur d'hydroxyde d'aluminium, et un revêtement extérieur d'acide isostéarique, ledit agent filmogène est le cyclopentasiloxane (et) un copolymère d'acrylate/diméthicone, et le diluant est l'isohexane/iso-dodécane/alkane en C$_{13}$ à C$_{15}$, ou

(iv) les particules de dioxyde de titane présentent un revêtement intérieur d'hydroxyde d'aluminium, et un revêtement extérieur d'acide isostéarique, ledit agent filmogène est le cyclopentasiloxane (et) un copolymère d'acrylate/diméthicone, et le diluant est le pentadécane, ou

(v) les particules de dioxyde de titane présentent un revêtement intérieur d'hydroxyde d'aluminium, et un revêtement extérieur d'acide isostéarique, ledit agent filmogène est le cyclopentasiloxane (et) un copolymère d'acrylate/diméthicone, et le diluant est le dodécane et tridécane, ou

(vi) les agents d'écran solaire sont l'oxyde de zinc, et des particules de dioxyde de titane, dans laquelle les particules de dioxyde de titane présentent un revêtement intérieur d'hydroxyde d'aluminium, et un revêtement extérieur d'acide isostéarique, ledit agent filmogène est le cyclopentasiloxane (et) un copolymère d'acrylate/diméthicone, et le diluant est l'isododécane, ou

(vii) les particules de dioxyde de titane présentent un revêtement intérieur d'hydroxyde d'aluminium, et un revêtement extérieur d'acide isostéarique, ledit agent filmogène est l'isododécane (et) un copolymère d'acrylate/diméthicone, et le diluant est l'isododécane, ou

(viii) les particules de dioxyde de titane présentent un revêtement intérieur d'hydroxyde d'aluminium, et un revêtement extérieur d'acide isostéarique, ledit agent filmogène est le cyclopentasiloxane (et) un copolymère d'acrylate/diméthicone, et le diluant est l'isododécane, et les agents d'écran solaire additionnels sont homosalate, octisalate, octocrylène, et avobenzone.

2. Composition d'écran solaire selon la revendication 1, dans lequel ledit dioxyde de titane est présent en une quantité de jusqu'à 40,0 % en poids, de préférence encore jusqu'à 30,0 % en poids, et le plus préférablement jusqu'à 20,0 % en poids.

3. Composition d'écran solaire selon l'une quelconque des revendications 1 ou 2, comprenant en outre un agent d'écran solaire additionnel choisi parmi l'oxyde de zinc et un ou plusieurs agents d'écran solaire organiques.

4. Composition d'écran solaire selon la revendication 3, dans lequel lesdits un ou plusieurs agents d'écran solaire organiques sont choisis dans le groupe consistant en acide p-aminobenzoïque et ses dérivés, butylméthoxy-dibenzoylméthane, benzophénones, benzophénones substitués par hydroxyle, benzophénones substitués par méthoxy, benzophénone-1, benzophénone-2, benzophénone-3, benzophénone-4, benzophénone-6, benzophénone-8, benzophénone-12, méthoxycinnamate, dihydroxypropyl-*p*-aminobenzoate d'éthyle, glycéryl-*p*-aminobenzoate, homosalate, anthranilate de méthyle, octocrylène, diméthyl-*p*-aminobenzoate d'octyle, méthoxycinnamate d'octyle, salicylate d'octyle, acide 2-phénylbenz-imidazole-5-sulfonique, salicylate de triéthanolamine, 3-(4-méthyl-benzylidène)-camphre, vaseline rouge, 3-(4-méthylbenzylidine)boran-2-one (méthylbenzidinecamphre), benzotriazole, salicylates, acide phényl-benzimidazole-5-sulfonique, méthylène-*bis*-benzotriazolyl-tétraméthyl-butyl-phénol, avobenzone, 4-isopropyldibenzoylméthane, octisalate, oxybenzone, bis-éthylhexyloxyphénol-méthoxy-triazine, octyltriéthoxy-silanol, triéthoxy-silane, et des combinaisons de ceux-ci.

5. Composition d'écran solaire selon l'une quelconque des revendications 1 à 4, dans laquelle ledit agent filmogène est présent en une quantité de 0,05 % en poids à 5,0 % en poids.

**6.** Composition d'écran solaire selon l'une quelconque des revendications 1 ou 2, comprenant en outre un agent d'écran solaire additionnel.

**7.** Dispositif, comprenant une partie applicatrice poreuse, et une mèche, et la composition d'écran solaire selon l'une quelconque des revendications 1 à 6.

**8.** Produit de consommation emballé, comprenant la composition d'écran solaire selon l'une quelconque des revendications 1 à 6.

**9.** Utilisation de la composition d'écran solaire selon l'une quelconque des revendications 1 à 6 pour protéger les cheveux contre le rayonnement UV.

**10.** Composition d'écran solaire selon l'une quelconque des revendications 1 à 6 à utiliser dans la protection de la peau contre le rayonnement UV.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 8545891 B